(19) Europäisches Patentamt
European Patent Office
Office européen des brevets

(11) EP 1 259 212 B1

(12) FASCICULE DE BREVET EUROPEEN

(45) Date de publication et mention de la délivrance du brevet:
**14.10.2009 Bulletin 2009/42**

(21) Numéro de dépôt: **01270305.4**

(22) Date de dépôt: **11.12.2001**

(51) Int Cl.:
***A61K 8/49*** *(2006.01)* ***A61K 8/97*** *(2006.01)*
***A61Q 1/02*** *(2006.01)*

(86) Numéro de dépôt international:
**PCT/FR2001/003937**

(87) Numéro de publication internationale:
**WO 2002/047627 (20.06.2002 Gazette 2002/25)**

# (54) COMPOSITION COSMETIQUE COLOREE TRANSPARENTE OU TRANSLUCIDE

GEFÄRBTE DURCHSICHTIGE ODER DURCHSCHEINENDE KOSMETISCHE ZUSAMMENSETZUNG

COLOURED TRANSPARENT OR TRANSLUCENT COSMETIC COMPOSITION

(84) Etats contractants désignés:
**AT BE CH CY DE DK ES FI FR GB GR IE IT LI LU MC NL PT SE TR**

(30) Priorité: **12.12.2000 FR 0016178**

(43) Date de publication de la demande:
**27.11.2002 Bulletin 2002/48**

(60) Demande divisionnaire:
**04027429.2 / 1 527 767**

(73) Titulaire: **L'ORÉAL**
**75008 Paris (FR)**

(72) Inventeurs:
- **JAGER-LEZER, Nathalie**
**F-91370 Verrieres-le-Buisson (FR)**
- **SIMON, Jean-Christophe**
**F-64665 Albach-Hahnlein (FR)**

(74) Mandataire: **Casalonga, Axel et al**
**Bureau Casalonga & Josse**
**Bayerstrasse 71/73**
**80335 München (DE)**

(56) Documents cités:

| | |
|---|---|
| **WO-A-99/24002** | **DE-A- 19 707 309** |
| **US-A- 3 937 811** | **US-A- 5 223 559** |
| **US-A- 5 750 125** | **US-A- 5 780 517** |

- **PATENT ABSTRACTS OF JAPAN vol. 1999, no. 06, 31 mars 1999 (1999-03-31) & JP 04 346909 A (PACIFIC CHEM IND CO), 2 décembre 1992 (1992-12-02)**

EP 1 259 212 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne des compositions cosmétiques transparentes ou translucides capables de déposer une couleur sur la peau, les lèvres ou les phanères, ainsi qu'un procédé pour les préparer.

L'apport de couleur sur la peau, les lèvres et les phanères, en particulier les cheveux, ongles et cils, est depuis toujours un important objet de recherche dans le domaine cosmétique et tout particulièrement dans le domaine du maquillage.

Cet apport de couleur se fait le plus souvent sous forme de pigments blancs ou colorés, éventuellement associés à des colorants, dans des bases cosmétiques donnant lieu à des dépôts colorés couvrants (rouge à lèvres, mascara, fard à paupières, eye-liner, vernis à ongles, fonds de teint) ou semi-transparents (fond de teint, fard à paupières, rouge à lèvres, vernis à ongles), l'effet recherché étant le plus souvent l'obtention d'une couleur intense ou le masquage des imperfections sous-jacentes.

Dans le domaine des fonds de teint par exemple, le masquage des imperfections cutanées par des produits couvrants ou semi-couvrants s'accompagne cependant presque toujours, malgré l'application en couche très fine, d'une certaine visibilité du dépôt et d'un aspect non naturel, le plus souvent indésirable.

Il existe par ailleurs des compositions cosmétiques, telles que des crèmes de soin, qui, après application en fine couche, sont parfaitement transparentes ou bien suffisamment translucides (voir WO 98/5234) pour conserver l'aspect naturel de la peau et ne masquer que légèrement les imperfections de celle-ci. Ces produits ne permettent cependant pas de colorer le substrat physiologique sur lequel elles sont déposées.

La demanderesse s'est fixé pour objectif de mettre au point une nouvelle gamme de produits de maquillage non couvrants permettant de déposer une couleur sur la peau, les lèvres ou les phanères tout en restant parfaitement "invisibles", c'est-à-dire des produits capables de donner des dépôts suffisamment transparents ou translucides pour conserver l'aspect naturel de la surface sous-jacente.

La présente invention a par conséquent pour objet des compositions cosmétiques colorées, transparentes ou translucides, pour le maquillage de la peau, des lèvres et des phanères, comprenant une base cosmétique transparente ou translucide dans la masse, et au moins un agent colorant en une quantité telle que la transmittance d'une couche de 10 $\mu$m de la composition finale, mesurée à la longueur d'onde du maximum d'un des pics d'absorption ou de diffusion de l'agent colorant, soit comprise entre 20 % et 80 %.

L'invention a également pour objet un procédé de préparation d'une composition cosmétique colorée transparente ou translucide décrite ci-dessus.

Les compositions cosmétiques objet de la présente invention permettent donc de colorer le substrat sur lequel elles sont appliquées tout en donnant, grâce à la grande "transparence" du dépôt obtenu, un aspect parfaitement naturel à la surface ainsi couverte.

Les compositions cosmétiques colorées de la présente invention ont pour caractéristique non seulement la capacité de coloration et la transparence du dépôt obtenu, mais également un aspect transparent ou translucide "dans la masse". Cette propriété de transparence ou de translucidité dans la masse signifie qu'une couche d'une épaisseur fixée arbitrairement à 1 cm, laisse passer une partie de la lumière visible soit en la diffusant (compositions translucides dans la masse) soit sans la diffuser (compositions transparentes dans la masse).

Cet aspect transparent ou translucide est très satisfaisant d'un point de vue esthétique et peut de ce fait être d'un grand intérêt commercial.

Les compositions cosmétiques de la présente invention sont caractérisées par le fait qu'elles ont une transmittance à 10 $\mu$m d'épaisseur, mesurée à la longueur d'onde du maximum d'un des pics d'absorption ou de diffusion de l'agent colorant, comprise entre 20 % et 80 %.

Cette épaisseur de couche de 10 $\mu$m à laquelle sont réalisées les mesures de transmittance des compositions de la présente invention a été choisie parce qu'elle correspond sensiblement à l'épaisseur d'un dépôt de maquillage obtenu par exemple avec un fond de teint ou un rouge à lèvres. Les valeurs obtenues par ces mesures rendent donc bien compte de ce que l'on appelle couramment le "rendu maquillage", c'est-à-dire de l'impression visuelle immédiate que donne la couche de maquillage.

La transmittance, telle que définie ici, est égale au rapport de l'intensité de lumière transmise par l'échantillon ($I_t$) à l'intensité de lumière transmise par le témoin ($I_0$) exprimé en %

$$T\ (\%) = I_t/I_0$$

Pour pouvoir réaliser des mesures sur une épaisseur de couche de 10 $\mu$m, la demanderesse utilise des porte-échantillons particuliers.

Il s'agit d'une lame transparente en verre ou en quartz dont la taille est fonction de la cellule de mesure du

spectrophotomètre utilisé (20 mm x 10 mm x 3 mm pour un CARY 300) présentant à sa surface un évidement plan d'une profondeur de 10 µm. Cet évidement plan est rempli avec l'échantillon et l'excédent est éventuellement arasé à l'aide d'une lame de manière à obtenir ainsi une couche parfaitement régulière d'une épaisseur de 10 µm.

Les mesures sont faites au moyen d'un spectrophotomètre à double faisceau UV-visible, modèle CARY 300 de la société Varian, en mode transmission et en utilisant comme témoin une lame transparente (en quartz ou en verre) d'épaisseur identique à celle recevant l'échantillon.

Comme indiqué ci-dessus, les valeurs de transmittance indiquées pour les compositions selon l'invention sont celles mesurées à la longueur d'onde correspondant au maximum de l'un des pics d'absorption (colorant) ou de diffusion (pigment) de l'agent colorant dans le domaine de la lumière visible ($\lambda$ = 400 à 750 nm).

L'erreur de mesure de la transmittance est de $\pm$ 5 %.

La transparence ou translucidité "dans la masse" des compositions cosmétiques colorées de la présente invention est évaluée visuellement pour une épaisseur de couche de 1 cm.

Les compositions cosmétiques colorées transparentes ou translucides sont obtenues grâce à l'association

- d'une base cosmétique, transparente ou translucide dans la masse et
- d'au moins un agent colorant approprié.
  Les bases cosmétiques pouvant être utilisées pour la préparation des compositions de la présente invention peuvent être constituées par toute base cosmétiquement acceptable remplissant les conditions de transparence ou de translucidité indispensables pour l'obtention des compositions cosmétiques colorées transparentes ou translucides.
  Ces conditions de transparence ou de translucidité sont
- une bonne transparence ou translucidité dans la masse de la base sans colorant, appréciée visuellement pour une épaisseur de 1 cm, et
- une transmittance de la base contenant le ou les colorant(s), mesurée pour une épaisseur de 10 µm à la longueur d'onde du maximum d'un des pics d'absorption ou de diffusion de l'agent colorant utilisé, comprise entre 20 % et 80%.

Il peut s'agir de phases hydrophiles ou lipophiles, de consistance liquide, épaissie, gélifiée, pâteuse ou solide.

De préférence, la base de la composition est sous forme d'un gel plus
ou moins rigide, aqueux ou huileux. Plus spécialement, ce gel est un gel rigide, présenté en coupelle ou en stick, de préférence en stick et sous forme anhydre.
En particulier, cette base est une base de rouge à lèvres ou de fond de teint anhydre.

La base huileuse contient une phase grasse liquide à température ambiante telle que celles classiquement utilisées en cosmétique. Cette phase grasse peut contenir des huiles polaires et/ou des huiles apolaires.

En particulier, les huiles polaires de l'invention sont :

- les huiles végétales hydrocarbonées à forte teneur en triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, ou de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule $R_aCOOR_b$ dans laquelle $R_a$ représente le reste d'un acide gras linéaire ou ramifié comportant de 1 à 40 atomes de carbone et $R_b$ représente une chaîne hydrocarbonée, notamment ramifiée, contenant de 1 à 40 atomes de carbone à condition que $R_a + R_b$ soit $\geq$ 10, comme par exemple l'huile de Purcellin (octanoate de cétostéaryle), l'isononanoate d'isononyle, le benzoate d'alcool en $C_{12}$ à $C_{15}$, le myristate d'isopropyle, le palmitate de 2-éthylhexyle, l'isostéarate d'isostéaryle, les octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en $C_8$ à $C_{26}$ comme l'alcool oléique ;
- les acides gras en $C_8$ à $C_{26}$ comme l'acide oléique, linolénique et linoléique ; et
- leurs mélanges.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle ou alcoxy, latéraux et/ou en bout de chaîne, groupements ayant chacun de 2 à 24 atomes de carbone ; les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyltriméthylsiloxysilicates ; les hydrocarbures linéaires ou ramifiés d'origine synthétique ou minérale, volatils ou non, tels que les huiles de paraffine volatiles (isoparaffines comme l'isododécane) ou non volatiles, et leurs dérivés, la vaseline, la lanoline liquide, les polydécènes, le polyisobutène hydrogéné tel que l'huile de Parléam, le squalane ou l'huile d'arara ; et leurs mélanges.

De préférence, les huiles sont des huiles apolaires et plus spécialement une huile ou un mélange d'huiles du type hydrocarboné d'origine minérale ou synthétique, choisies en particulier parmi les alcanes comme l'huile de Parléam, les isoparaffines comme l'isododécane, le squalane et leurs mélanges. Avantageusement, ces huiles sont associées à une ou plusieurs huiles de silicones phénylées.

De préférence, la phase grasse liquide contient au moins une huile non volatile choisie en particulier parmi les huiles hydrocarbonées d'origine minérale, végétale ou synthétique, les esters ou éthers de synthèse, les huiles de silicone et leurs mélanges.

La phase grasse liquide totale représente, en pratique, de 5 à 99,95 %, de préférence de 10 à 80%, et mieux de 20 à 75 % du poids total de la composition.

Cette phase grasse est avantageusement structurée par un gélifiant de phase grasse comme

(a) les polyamides gélifiants, notamment de masse moléculaire inférieure à 100 000 et de préférence inférieure à 50 000, par exemple de masse moléculaire allant de 2000 à 20 000, comportant éventuellement des groupements alkyle latéraux ou en bout de chaîne, ayant de 8 à 120 atomes de carbone et de préférence de 12 à 60 atomes de carbone,
(b) les galactomannanes hydrophobes comportant notamment de 1 à 6, et de préférence de 2 à 4 groupes OH, par motif ose, substitués par un groupe alkyle en $C_{1-6}$, de préférence en $C_{1-3}$,
(c) les silices hydrophobes pyrogénées,
(d) et les associations de ces agents gélifiants.

Les polyamides gélifiants sont par exemple les résines polyamides résultant de la condensation d'un acide dicarboxylique aliphatique et d'une diamine, incluant les composés ayant plus de 2 groupes carboxyle et plus de 2 groupes amine, les groupes carboxyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid® 930, 744 ou 1655) ou par la société Olin Mathieson Chemical Corp., sous la marque Onamid® notamment Onamid® S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Les polyamides peuvent être aussi ceux résultant d'une polycondensation entre un diacide carboxylique comportant au moins 32 atomes de carbone (notamment de 32 à 44 atomes de carbone) et une diamine ayant au moins 2 atomes de carbone (notamment de 2 à 36 atomes de carbone). Le diacide est de préférence un dimère d'acide gras ayant au moins 16 atomes de carbone comme l'acide oléique, linoléique ou linolénique. La diamine est de préférence l'éthylènediamine, l'hexylènediamine ou l'hexaméthylènediamine. Si les polymères comportent un ou deux groupements acide carboxylique terminaux, il est avantageux de les estérifier par un monoalcool ayant au moins 4 atomes de carbone, de préférence de 10 à 36 atomes de carbone, mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (I) suivante :

(I) 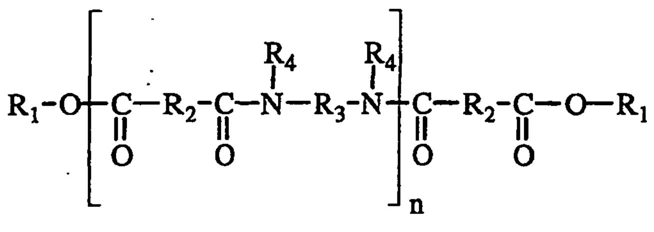

dans laquelle n désigne un nombre entier de motifs amide tel que le nombre de groupes ester représente de 10 % à 50

% du nombre total des groupes ester et amide ; chacun des symboles R1 désigne indépendamment un groupe alkyle ou alcényle ayant au moins 4 atomes de carbone et notamment de 4 à 24 atomes de carbone ; chacun des symboles $R_2$ représente indépendamment un groupe hydrocarboné en $C_4$ à $C_{42}$ à condition que 50 % des groupes $R_2$ représentent un groupe hydrocarboné en $C_{30}$à $C_{42}$ ; chacun des symboles $R_3$ représente indépendamment un groupe organique pourvu d'au moins 2 atomes de carbone, d'atomes d'hydrogène et optionnellement d'un ou plusieurs atomes d'oxygène ou d'azote ; et chacun des symboles $R_4$ représente indépendamment un atome d'hydrogène, un groupe alkyle en $C_1$ à $C_{10}$ ou une liaison directe à $R_3$ ou à un autre $R_4$ de sorte que l'atome d'azote auquel sont liés à la fois $R_3$ et $R_4$ fasse partie d'une structure hétérocyclique définie par $R_4$-N-$R_3$, avec au moins 50 % des $R_4$ représentant un atome d'hydrogène.

Dans le cas particulier de la formule (I), les chaînes grasses terminales éventuellement fonctionnalisées au sens de l'invention sont des chaînes terminales liées au dernier hétéroatome, ici l'azote, du squelette polyamide.

En particulier, les groupes ester de la formule (I), qui font partie des chaînes grasses terminales et/ou latérales au sens de l'invention, représentent de 15 à 40 % du nombre total des groupes ester et amide et mieux de 20 à 35 %. De plus, n représente avantageusement un nombre entier allant de 1 à 5 et de préférence supérieur à 2.

De préférence, $R_1$ est un groupe alkyle en $C_{12}$ à $C_{22}$ et plus préférentiellement en $C_{16}$ à $C_{22}$. Avantageusement, $R_2$ peut être un groupe hydrocarboné (alkylène) en $C_{10}$ à $C_{42}$. De préférence, 50 % au moins et plus préférentiellement au moins 75 % des symboles $R_2$ sont des groupes ayant de 30 à 42 atomes de carbone. Les autres symboles $R_2$ sont des groupes hydrogénés en $C_4$ à $C_{19}$ et même en $C_4$ à $C_{12.}$ De préférence, $R_3$ représente un groupe hydrocarboné en $C_2$ à $C_{36}$ ou un groupe polyoxyalkyléné et $R_4$ représente un atome d'hydrogène. Plus préférentiellement, $R_3$ représente un groupe hydrocarboné en $C_2$ à $C_{12.}$

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Selon l'invention, la structuration de la phase grasse liquide est obtenue de préférence à l'aide d'un ou plusieurs polymères de formule (I). En général, les polymères de formule (I) se présentent sous forme de mélanges de polymères, ces mélanges pouvant en outre contenir un produit de synthèse correspondant à un composé de formule (I) où n vaut 0, c'est-à-dire un diester.

Ces polymères présentent du fait de leur (s) chaîne (s) grasse (s), une bonne solubilité dans les huiles et donc conduisent à des compositions macroscopiquement homogènes même avec un taux élevé (au moins 25%) de polymère, contrairement à des polymères exempts de chaîne grasse.

Comme polymères structurants préférés de formule (I) utilisables dans l'invention, on peut citer les polyamides modifiés par des chaînes grasses latérales et/ou des chaînes grasses terminales ayant de 8 à 120 atomes de carbone et notamment de 12 à 68 atomes de carbone, les chaînes grasses terminales étant liées au squelette polyamide par des groupes ester. De préférence, ces polymères comportent une chaîne grasse à chaque extrémité du squelette polymérique et en particulier du squelette polyamide.

A titre d'exemples de polyamides structurants de formule (I) utilisables dans la composition selon l'invention, on peut citer les produits commerciaux vendus par la société Arizona Chemical sous les noms Uniclear® 80 et Uniclear® 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymères d'un diacide en $C_{36}$ condensé sur l'éthylènediamine, de masse moléculaire moyenne en poids respectivement d'environ 600 ou 4000. Les groupes ester terminaux résultent de l'estérification des terminaisons d'acide restantes par l'alcool cétylique ou stéarylique ou leurs mélanges (appelés aussi alcool cétylstéarylique).

Les galactomannanes sont en particulier des dérivés éthylés de guar ayant notamment un degré de substitution de 2 à 3, tels que ceux commercialisés par la société AQUALON sous les dénominations N-Hance-AG-200® ou N-Hance-AG-50®.

La silice pyrogénée présente de préférence une taille de particules pouvant être nanométrique à micrométrique, par exemple allant d'environ de 5 à 200 nm.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations AEROSIL 130®, "AEROSIL 200®, AEROSIL 255®, AEROSIL 300®, AEROSIL 380® par la société Degussa, ou sous les dénominations CAB-O-SIL HS-5®, "CAB-O-SIL EH-5®, CAB-O-SIL LM-130®, CAB-O-SIL MS-55®, et CAB-O-SIL M-5® par la société Cabot.

Il est possible de modifier chimiquement la surface de ladite silice, par une réaction chimique réduisant le nombre de groupes silanol. On peut notamment substituer des groupes silanol par des groupements hydrophobes et obtenir ainsi une silice hydrophobe. Les groupements hydrophobes peuvent être :

- des groupements triméthylsiloxy, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous la dénomination AEROSIL R812® par la société Degussa, et

sous la dénomination CAB-O-SIL TS-530® par la société Cabot.

- des groupements diméthylsilyloxy ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les dénominations AEROSIL R972® et AEROSIL R974® par la société Degussa, et sous les dénominations CAB-O-SIL TS-610® et CAB-O-SIL TS-720® par la société Cabot.
- des groupements issus de la réaction de la silice pyrogénée avec des alcoxysilanes ou des siloxanes. Ces silices traitées sont par exemple celles vendues sous la référence AEROSIL R805® par la société Degussa.

Lorsque le gel est un gel aqueux, on peut utiliser tout gélifiant de phase aqueuse du type dérivé de cellulose tel que l'hydroxyéthylcellulose et la carboxyméthylcellulose ou dérivé acrylique comme les copolymères d'acide acrylique et d'acrylates d'alkyle en $C_{10-30}$, réticulés, par exemple la série des PENIULEN® et le CARBOPOL®980 commercialisés par la société GOODRICH, les dérivés d'argile du type sodium-magnésium silicate comme la LAPONITE XLS ou XLG commercialisée par la société LAPORTE et les associations de ces gélifiants. Le gel aqueux peut être un gel à base d'eau ou à base d'un mélange hydroalcoolique.

Le gélifiant représente de 0,05 à 90 % en poids, de préférence de 2 à 60 % en poids et en particulier de 5 à 40 % en poids, du poids total de la composition cosmétique colorée.

Les bases cosmétiques transparentes ou translucides utilisées selon l'invention sont de préférence sensiblement incolores.

Dans ces bases cosmétiques transparentes ou translucides, on introduit selon la présente invention, un ou plusieurs agents colorants.

Selon la présente invention, le terme "agent colorant" englobe notamment les colorants hydrosolubles ou liposolubles, les pigments, les nacres, les laques et leurs mélanges.

On peut citer à titre de colorants hydrosolubles, les colorants synthétiques tels que la fuschine, les extraits de plantes tels que les extraits de sorgho, de *pterocarpus soyauxii,* de *monascus,* de *lawsonia inermis,* de *mercurialis perenis, d'helianthus aanus, d'impatiens balsamina,* de *curcuma longa,* de *phytolacca décandra,* de *solidago aureus,* de *juglans regia, d'iris germanica, d'alkanna tinctoria,* de *chrozophora tinctoria, d'isatis tinctoria,* et les mélanges de ces colorants.

Les colorants liposolubles sont par exemple le Rouge Soudan III (CTFA : D&C Red 17), la lutéine, le vert de quinizarine (CTFA : D&C green 6), le pourpre d'alizurol SS (CTFA : D&C violet n° 2), les dérivés des caroténoïdes tels que le lycopène, le bétacarotène, la bixine ou la capsantéine, les dérivés de rocou et de fuschine (voir exemple 2) , et leurs mélanges.

Un certain nombre de ces colorants tels que les extraits de *pterocarpus soyauxii,* de *monascus* et de *lawsonia inermis,* ont une forte affinité pour la peau et peuvent ainsi lui conférer une coloration semi-permanente, c'est-à-dire une coloration résistant à plusieurs lavages.

Par pigments, il faut comprendre des particules blanches ou colorées, minérales ou organiques, enrobées ou non. On peut citer par exemple les dioxydes de titane, de zirconium ou de cérium, les oxydes de zinc, de fer ou de chrome, le bleu ferrique, l'hydrate de chrome, le noir de carbone, les outremers (polysulfures d'aluminosilicates), le violet de manganèse, le pyrophosphate de manganèse et certaines poudres métalliques telles que les poudres d'argent ou d'aluminium, et leurs mélanges.

Par nacres, on entend des pigments nacrés blancs tels que le mica recouvert d'oxyde de titane ou d'oxychlorure de bromuth et des pigments nacrés colorés tels que le micatitane recouvert avec des oxydes de fer, du bleu ferrique ou de l'oxyde de chrome, ou avec un pigment organique type précipité.

Les laques utilisables dans les compositions de la présente invention sont par exemple les laques à base de carmin de cochenille ou à base de sels de calcium, de baryum, d'aluminium, de strontium ou de zirconium, de colorants acides, et leurs mélanges.

La quantité d'agent colorant est déterminante pour la présente invention. En effet, cette quantité détermine directement la transmittance de la composition qui, pour une épaisseur de 10 $\mu$m, doit être comprise entre 20 % et 80 % à la longueur d'onde correspondant au maximum de l'un des pics d'absorption ou de diffusion de l'agent colorant.

En dessous d'une certaine quantité d'agent colorant (transmittance à 10 $\mu$m supérieure à 80 %), la composition donnera lieu à un dépôt suffisamment transparent ou translucide pour préserver l'aspect naturel de la peau, des lèvres ou des phanères, mais elle ne permettra pas d'apporter une coloration visible à l'oeil nu.

Au contraire, pour une proportion d'agent colorant trop importante (transmittance à 10 $\mu$m inférieure à 20 %), la couleur du dépôt de maquillage sera certes visible mais la transparence ou la translucidité de celui-ci sera insuffisante pour préserver l'aspect naturel de la peau. Par ailleurs, des compositions cosmétiques contenant une proportion trop importante d'agent colorant seront insuffisamment transparentes ou translucides dans la masse.

Dans un mode de réalisation préféré de l'invention, les compositions cosmétiques colorées ont une transmittance, mesurée pour une épaisseur de 10 $\mu$m, à la longueur d'onde correspondant au maximum de l'un des pics

d'absorption ou de diffusion de l'agent colorant, comprise entre 25 % et 80 %.

Le procédé de détermination de la quantité d'agent colorant appropriée permettant d'obtenir des compositions colorées transparentes ou translucides selon la présente invention sera décrit plus en détail ci-dessous.

La quantité appropriée d'agent colorant dépendra évidemment de ses propriétés physicochimiques telles que sa solubilité dans la base cosmétique, sa granulométrie ou son coefficient d'absorption molaire (ε).

Les compositions cosmétiques colorées transparentes ou translucides selon la présente invention contiennent généralement de 0,05 % à 3 % en poids et de préférence de 0,1 à 1% en poids d'agent(s) colorant(s), sur la base du poids total de la composition cosmétique colorée. Pour les nacres, on peut aller jusqu'à 3% en poids ; pour les pigments, les laques ou les colorants, on ne va de préférence que jusqu'à 1% en poids

Dans un mode de réalisation préféré de la présente invention, la composition cosmétique colorée contient au moins un colorant hydrosoluble
ou liposoluble, soluble dans la base cosmétique.

Dans un autre mode de réalisation particulièrement intéressant de l'invention, la composition cosmétique colorée contient, en tant qu'agent(s) colorant(s), uniquement un ou plusieurs colorants solubles dans la base cosmétique, et est exempte d'agents colorants insolubles de type pigments, nacres ou laques.

Dans un mode de réalisation plus particulièrement préféré de la présente invention, la base cosmétique est une base lipophile contenant un ou plusieurs colorants lipophiles solubles dans celle-ci.

De telles compositions contenant uniquement des colorants solubles ont en effet un bon pouvoir colorant associé à d'excellentes propriétés de transparence dues à l'absence de diffusion de la lumière par des particules insolubles.

La présente invention a également pour objet un procédé de préparation des compositions cosmétiques colorées transparentes ou translucides de la présente invention qui présente pour principales caractéristiques :

- le choix d'une base cosmétique transparente ou translucide appropriée et
- le dosage du ou des agent(s) colorant(s), c'est-à-dire l'incorporation d'une quantité appropriée d'agent(s) colorant(s) permettant de résoudre le problème technique à l'origine de l'invention, c'est-à-dire l'obtention d'un dépôt coloré ayant une transmittance (à 10 μm et à $\lambda_{max}$) comprise entre 20 % et 80 %.

La détermination de la quantité appropriée d'agent colorant comprend les étapes consistant :

(a) à sélectionner une base cosmétique transparente ou translucide telle que décrite ci-dessus,
(b) à préparer une série d'échantillons de cette base cosmétique transparente
ou translucide contenant des quantités croissantes d'un agent colorant dissous ou dispersé dans ladite base cosmétique,
(c) à étaler chacun des échantillons ainsi préparés sur une lame transparente présentant un évidement d'une profondeur de 10 μm,
(d) éventuellement à araser l'excédent de l'échantillon de manière à obtenir une couche d'une épaisseur de 10 μm,
(e) à mesurer pour chacun des échantillons la transmittance de ladite couche à la longueur d'onde correspondant au maximum de l'un des pics d'absorption ou de diffusion de l'agent colorant, et
(f) à tracer la courbe d'étalonnage transmittance = f(concentration de l'agent colorant).

On prépare ensuite des compositions cosmétiques colorées en incorporant dans une base cosmétique transparente ou translucide identique à
ou différente de celle sélectionnée dans l'étape (a) ci-dessus et se trouvant à l'état liquide, un ou plusieurs agents colorants chacun en une quantité donnant, d'après la courbe d'étalonnage réalisée pour chaque agent colorant, une transmittance (à 10 μm) comprise entre 20 % et 80 %, de préférence entre 25 % et 80 %.

Pour recevoir l'agent colorant, la base cosmétique doit bien entendu être à l'état liquide. La consistance liquide peut être une propriété de la base en tant que telle à température ambiante, ou elle peut être le résultat de la fusion ou dissolution d'une base cosmétique solide à température ambiante.

Les bases cosmétiques anhydres solides préférées selon la présente invention sont de préférence liquéfiées par fusion à une température faiblement supérieure à leur point de fusion.

La présente invention est illustrée à l'aide des exemples ci-après.

__Exemple 1__

| Rouge à lèvres | |
|---|---|
| Uniclear® 100 | 25 % |
| Octyldodécanol | 10 % |
| Rocou® | 0,2 % (matière active colorante) |
| Huile de Parléam | q.s.p. 100 % en poids |

Uniclear® 100 : condensat d'un diacide en $C_{36}$ hydrogéné et d'éthylènediamine, estérifié par l'alcool stéarylique (masse molaire moyenne en poids environ 4000) commercialisé par la société ARIZONA CHEMICAL.
Rocou® : solution à 4 % de graines de rocou dans de l'huile de soja (CI : 75120) commercialisée par la société WARNER-JENKINSON.
Dans un poêlon, on introduit l'Uniclear® 100 et les huiles. On met l'ensemble sous agitation magnétique et on le chauffe dans un premier temps à 100°C pour amener l'Uniclear à l'état liquide. Puis, on continue à chauffer jusqu'à la température nécessaire pour obtenir un liquide transparent homogène. On se place alors à 10°C au-dessus de cette température. On introduit dans le mélange le colorant et on homogénéise l'ensemble sous agitation magnétique pendant 1 heure. On coule la composition dans un moule chauffé à 45°C pour former un stick qu'on place, après un début de prise en masse, au congélateur pendant 15 minutes (-21°C).
La composition obtenue a un aspect translucide dans la masse (1 cm) et donne lieu à un dépôt parfaitement transparent de couleur orange ayant une transmittance à 498 nm (λmax du colorant) et à 10 μm d'épaisseur égale à 78 %.

__**Exemple 2**__

| Rouge à lèvres | |
|---|---|
| Uniclear® 100 | 25 % |
| Octyldodécanol | 10 % |
| Complexe MMB Red® 33/3 | 0,2 % (matière.active colorante) |
| Huile de Parléam | q.s.p. 100 % en poids |

Uniclear® 100 : condensat d'un diacide en $C_{36}$ hydrogéné et d'éthylènediamine, estérifié par l'alcool stéarylique (masse molaire moyenne en poids environ 4000) commercialisé par la société ARIZONA CHEMICAL.
Complexe MMB Red® 33/3 : colorant commercialisé sous cette dénomination par la société PHYTOCOS et désignant le mélange : sel disodique de fuschine acide D/palmitate myristate de lysine/dipropylèneglycol/acide benzoïque/phénoxyéthanol/solution à 3 % de D&C Red n° 33 (CI 17200)/conservateurs : p-hydroxybenzoate de méthyle, butyle, éthyle, propyle.
On prépare un stick par le même procédé que dans l'exemple 1.
La composition obtenue a un aspect translucide dans la masse (1 cm) et donne lieu à un dépôt parfaitement transparent de couleur rose fuschia ayant une transmittance à 530 nm ($\lambda_{max}$ du colorant) et à 10 μm d'épaisseur égale à 40 %.

**Revendications**

1. Composition cosmétique colorée, transparente ou translucide, pour le maquillage de la peau, des lèvres et des phanères, comprenant une base cosmétique transparente ou translucide dans la masse, et au moins un agent colorant en une quantité telle que la transmittance d'une couche de 10 μm de la composition finale, mesurée à la longueur d'onde du maximum de l'un des pics d'absorption ou de diffusion de l'agent colorant, soit comprise entre 20 % et 80 %.

2. Composition cosmétique colorée selon la revendication 1, **caractérisée par le fait que** la base cosmétique transparente ou translucide est une base sensiblement incolore.

3. Composition cosmétique colorée selon la revendication 1 ou 2, **caractérisée par le fait que** la base cosmétique transparente ou translucide est choisie parmi les gels aqueux ou huileux, notamment sous forme de sticks.

**4.** Composition cosmétique colorée selon l'une quelconque des revendications précédentes, **caractérisée par le fait que** la base est un gel anhydre formé d'une phase grasse liquide à température ambiante comprenant des huiles polaires et/ou apolaires structurée par un gélifiant de phase grasse choisi parmi les silices pyrogénées hydrophobes, les polyamides gélifiants, les galactomannanes hydrophobes, et leurs mélanges.

**5.** Composition cosmétique colorée selon la revendication 4,
**caractérisée par le fait que** les polyamides gélifiants correspondent à la formule (I) :

(I)

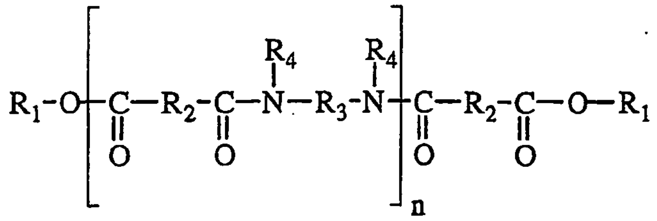

dans laquelle n désigne un nombre entier de motifs amide tel que le nombre de groupes ester représente de 10 % à 50 % du nombre total des groupes ester et amide ; chacun des symboles $R_1$ désigne indépendamment un groupe alkyle ou alcényle ayant au moins 4 atomes de carbone et notamment de 4 à 24 atomes de carbone ; chacun des symboles $R_2$ représente indépendamment un groupe hydrocarboné en $C_4$ à $C_{42}$ à condition que 50 % des groupes $R_2$ représentent un groupe hydrocarboné en $C_{30}$ à $C_{42}$ ; chacun des symboles $R_3$ représente indépendamment un groupe organique pourvu d'au moins 2 atomes de carbone, d'atomes d'hydrogène et optionnellement d'un ou plusieurs atomes d'oxygène ou d'azote ; et chacun des symboles $R_4$ représente indépendamment un atome d'hydrogène, un groupe alkyle en $C_1$ à $C_{10}$ ou une liaison directe à $R_3$ ou à un autre $R_4$ de sorte que l'atome d'azote auquel sont liés à la fois $R_3$ et $R_4$ fasse partie d'une structure hétérocyclique définie par $R_4$-N-$R_3$, avec au moins 50 % des $R_4$ représentant un atome d'hydrogène..

**6.** Composition cosmétique colorée selon l'une quelconque des revendications précédentes, **caractérisée par le fait que** le ou les agent(s) colorant(s) est (ou sont) choisi(s) parmi les colorants hydrosolubles, les colorants liposolubles, les pigments, les nacres, les laques et leurs mélanges.

**7.** Composition cosmétique colorée selon la revendication 6,
**caractérisée par le fait que** les colorants hydrosolubles sont choisis parmi es extraits de sorgho, *de pterocarpus soyauxii,* de *monascus,* de *lawsonia inermis,* de *mercurialis perenis, d'helianthus aanus, d'impatiens balsamina,* de *curcuma longa,* de *phytolacca décandra, de solidago aureus, de juglans regia, d'iris germanica, d'alkanna tinctoria,* de *chrozophora tinctoria,* d'*isatis tinctoria* et leurs mélanges.

**8.** Composition cosmétique colorée selon la revendication 6,
**caractérisée par le fait que** les colorants liposolubles sont choisis parmi le Rouge Soudan III, la lutéine, le vert de quinizarine, le pourpre d'alizurol SS, les dérivés des caroténoïdes tels que le lycopène, le bétacarotène, la bixine ou la capsantéine, et les dérivés de rocou et de fuschine, et leurs mélanges.

**9.** Composition cosmétique colorée selon la revendication 6,
**caractérisée par le fait que** les pigments sont des pigments blancs ou colorés, minéraux ou organiques, enrobés ou non, choisis parmi les dioxydes de titane, de zirconium ou de cérium, les oxydes de zinc, de fer ou de chrome, le bleu ferrique, l'hydrate de chrome, le noir de carbone, les outremers, le violet de manganèse, le pyrophosphate de manganèse et certaines poudres métalliques telles que les poudres d'argent ou d'aluminium, et leurs mélanges.

**10.** Composition cosmétique colorée selon la revendication 6,
**caractérisée par le fait que** les nacres sont choisies parmi le mica recouvert d'oxyde de titane ou d'oxychlorure de bismuth et le micatitane recouvert avec des oxydes de fer, du bleu ferrique ou de l'oxyde de chrome, ou avec un pigment organique précipité.

**11.** Composition cosmétique colorée selon la revendication 6,
**caractérisée par le fait que** les laques sont choisies parmi les laques à base de carmin de cochenille ou à base de sels de calcium, de baryum, d'aluminium, de strontium ou de zirconium, de colorants acides, et leurs mélanges.

**12.** Composition cosmétique colorée selon la revendication 6, **caractérisée par le fait qu'**elle contient au moins un colorant hydrosoluble ou liposoluble, soluble dans la base cosmétique.

**13.** Composition cosmétique colorée selon la revendication 12, **caractérisée par le fait qu'**elle contient, en tant qu'agent(s) colorant(s), uniquement un ou plusieurs colorants solubles dans la base cosmétique et qu'elle est exempte d'agents colorants insolubles de type pigments, nacres ou laques.

**14.** Composition cosmétique colorée selon la revendication 12 ou 13, **caractérisé par le fait que** la base cosmétique est une base lipophile et qu'elle contient un ou plusieurs colorants lipophiles solubles dans celle-ci.

**15.** Composition cosmétique colorée selon l'une quelconque des revendications précédentes, **caractérisée par le fait que** l'agent colorant est présent en une quantité telle que la transmittance d'une couche de 10 μm de la composition finale, mesurée à la longueur d'onde du maximum de l'un des pics d'absorption ou de diffusion de l'agent colorant, soit comprise entre 25 % et 80%.

**16.** Composition cosmétique colorée selon l'une quelconque des revendications précédentes, **caractérisée par le fait que** la quantité d'agent(s) colorant(s) est comprise entre 0,05 % et 3 % en poids, et de préférence entre 0,1 et 1% en poids d'agent colorant, par rapport au poids total de la composition cosmétique colorée.

**17.** Composition cosmétique colorée selon l'une quelconque des revendications précédentes, **caractérisée par le fait qu'**elle se présente sous forme de rouge à lèvres anhydre ou de fond de teint anhydre.

**18.** Procédé de préparation d'une composition cosmétique colorée transparente ou translucide selon l'une quelconque des revendications 1 à 17, **caractérisé par le fait qu'**il comprend les étapes consistant

(a) à sélectionner une base cosmétique transparente ou translucide,
(b) à préparer une série d'échantillons de cette base cosmétique transparente
ou translucide contenant des quantités croissantes d' un agent colorant dissous ou dispersé dans ladite base cosmétique,
(c) à étaler chacun des échantillons ainsi préparés sur une lame transparente présentant un évidement d'une profondeur de 10 μm
(d) éventuellement à araser l'excédent de l'échantillon de manière à obtenir une couche régulière d'une épaisseur de 10 μm,
(e) à mesurer pour chacun des échantillons la transmittance de ladite couche à la longueur d'onde correspondant au maximum de l'un des pics d'absorption ou de diffusion de l'agent colorant,
(f) à tracer la courbe d'étalonnage transmittance = f (concentration de l'agent colorant), et
(g) à incorporer dans une base cosmétique transparente ou translucide identique à ou différente de celle sélectionnée dans l'étape (a) ci-dessus et se trouvant à l'état liquide, au moins un agent colorant, chacun en une quantité donnant, d'après la courbe d'étalonnage réalisée pour chaque agent colorant, une transmittance (à 10 μm) comprise entre 20 % et 80 %, de préférence entre 25 % et 80 %.

## Claims

**1.** Transparent or translucent coloured cosmetic composition for making up the skin, lips and superficial body growths comprising a bulk transparent or translucent cosmetic base and at least one colouring agent in an amount such that the transmission of a 10 μm layer of the final composition, measured at the wavelength of the maximum of one of the absorption or scattering peaks of the colouring agent, is between 20% and 80%.

**2.** Coloured cosmetic composition according to Claim 1, **characterized in that** the transparent or translucent cosmetic base is a substantially colourless base.

**3.** Coloured cosmetic composition according to Claim 1 or 2, **characterized in that** the transparent or translucent cosmetic base is chosen from aqueous or oily gels, in particular in the form of sticks.

**4.** Coloured cosmetic composition according to any one of the preceding claims, **characterized in that** the base is an anhydrous gel formed from a fatty phase which is liquid at ambient temperature comprising polar and/or nonpolar oils, which fatty phase is structured by a gelling agent for fatty phases chosen from hydrophobic pyrogenic silicas, gelling polyamides, hydrophobic galactomannans, and their mixtures.

**5.** Coloured cosmetic composition according to Claim 4, **characterized in that** the gelling polyamides correspond to the formula (I):

(I) 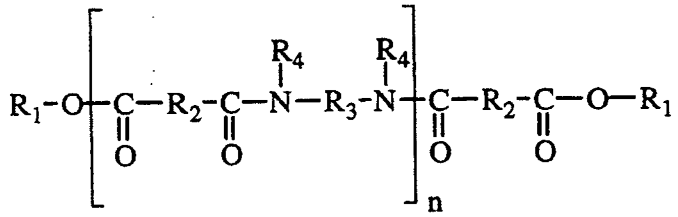

in which n denotes a whole number of amide units such that the number of ester groups represents from 10% to 50% of the total number of the ester and amide groups; each of the $R_1$ symbols independently denotes an alkyl or alkenyl group having at least 4 carbon atoms and in particular from 4 to 24 carbon atoms; each of the $R_2$ symbols independently represents a $C_4$ to $C_{42}$ hydrocarbon group, provided that 50% of the $R_2$ groups represent a $C_{30}$ to $C_{42}$ hydrocarbon group; each of the $R_3$ symbols independently represents an organic group provided with at least 2 carbon atoms, with hydrogen atoms and optionally with one or more oxygen or nitrogen atoms; and each of the $R_4$ symbols independently represents a hydrogen atom, a $C_1$ to $C_{10}$ alkyl group or a direct bond to $R_3$ or to another $R_4$, so that the nitrogen atom to which both $R_3$ and $R_4$ are bonded forms part of a heterocyclic structure defined by $R_4$-N-$R_3$, with at least 50% of the $R_4$ groups representing a hydrogen atom.

**6.** Coloured cosmetic composition according to any one of the preceding claims, **characterized in that** the colouring agent or agents is (or are) chosen from water-soluble dyes, fat-soluble dyes, pigments, pearlescent agents, lakes and their mixtures.

**7.** Coloured cosmetic composition according to Claim 6, **characterized in that** the water-soluble dyes are chosen from extracts of sorghum, of *Pterocarpus soyauxii,* of *Monascus,* of *Lawsonia inermis,* of *Mercurialis perenis,* of *Helianthus* aanus, of *Impatiens balsamina,* of *Curcuma longa,* of *Phytolacca decandra,* of *Solidago aureus,* of *Juglans regia,* of *Iris germanica,* of Alkanna *tinctoria,* of *Chrozophoro tinctoria* or of *Isatis* tinctoria, and their mixtures.

**8.** Coloured cosmetic composition according to Claim 6, **characterized in that** the fat-soluble dyes are chosen from Sudan red III, lutein, quinizarin green, alizurol purple SS, carotenoid derivatives, such as lycopene, β-carotene, bixin or capsantein, annatto and fuchsin derivatives, and their mixtures.

**9.** Coloured cosmetic composition according to claim 6, **characterized in that** the pigments are white or coloured, inorganic or organic and coated or uncoated pigments chosen from titanium, zirconium or cerium dioxides, zinc, iron or chromium oxides, ferric blue, chromium hydrate, carbon black, ultramarines, manganese violet, manganese pyrophosphate and some metal powders, such as silver or aluminium powders, and their mixtures.

**10.** Coloured cosmetic composition according to Claim 6, **characterized in that** the pearlescent agents are chosen from mica covered with titanium oxide or with bismuth oxychloride and titanium oxide-coated mica covered with iron oxides, ferric blue or chromium oxide or with a precipitated organic pigment.

**11.** Coloured cosmetic composition according to Claim 6, **characterized in that** the lakes are chosen from lakes based on cochineal carmine or based on calcium, barium, aluminium, strontium or zirconium salts, on acid dyes, and their mixtures.

**12.** The coloured cosmetic composition according to Claim 6, **characterized in that** it comprises at least one water-soluble or fat-soluble dye which is soluble in the cosmetic base.

**13.** Coloured cosmetic composition according to Claim 12, **characterized in that** it comprises, as colouring agent(s),

solely one or more dyes which are soluble in the cosmetic base and **in that** it is devoid of insoluble colouring agents of pigment, pearlescent agent or lake type.

**14.** Coloured cosmetic composition according to Claim 12 or 13, **characterized in that** the cosmetic base is a lipophilic base and **in that** it comprises one or more lipophilic dyes which are soluble in the latter.

**15.** Coloured cosmetic composition according to any one of the preceding claims, **characterized in that** the colouring agent is present in an amount such that the transmission of a 10 μm layer of the final composition, measured at the wavelength of the maximum of one of the absorption or scattering peaks of the colouring agent, is between 25% and 80%.

**16.** Coloured cosmetic composition according to any one of the preceding claims, **characterized in that** the amount of colouring agent(s) is between 0.05% and 3% by weight and preferably between 0.1 and 1% by weight with respect to the total weight of the coloured cosmetic composition.

**17.** Coloured cosmetic composition according to any one of the preceding claims, **characterized in that** it is provided in the form of an anhydrous lipstick or of an anhydrous foundation.

**18.** Process for the preparation of a transparent or translucent coloured cosmetic composition according to any one of Claims 1 to 17, **characterized in that** it comprises the stages consisting

(a) in selecting a transparent or translucent cosmetic base,
(b) in preparing a series of samples of this transparent or translucent cosmetic base comprising increasing amounts of a colouring agent dissolved or dispersed in the said cosmetic base,
(c) in spreading each of the samples thus prepared over a transparent slide exhibiting a recess with a depth of 10 μm,
(d) in optionally leveling out the excess of the sample, so as to obtain an even layer with a thickness of 10 μm,
(e) in measuring, for each of the samples, the transmission of the said layer at the wavelength corresponding to the maximum of one of the absorption or scattering peaks of the colouring agent,
(f) in plotting the transmission = f(concentration of the colouring agent) calibration curve, and
(g) in incorporating at least one colouring agent in a transparent or translucent cosmetic base which is identical to or different than that selected in stage
(a) above and which is in the liquid state, each of the colouring agents being incorporated in an amount giving, from the calibration curve prepared for each colouring agent, a transmission (at 10 μm) of between 20% and 80%, preferably between 25% and 80%.

## Patentansprüche

**1.** Durchsichtige oder durchscheinende, farbige kosmetische Zusammensetzung zum Schminken der Haut, der Lippen und der Hautanhangsgebilde, die eine in der Masse durchsichtige oder durchscheinende kosmetische Basisformulierung und mindestens ein Farbmittel in einer solchen Menge enthält, dass die Durchlässigkeit einer Schicht der fertigen Zusammensetzung von 10 μm, die bei einer Wellenlänge, die dem Maximum eines der Peaks der Absorption oder der Streuung des Farbmittels entspricht, gemessen wird, im Bereich von 20 bis 80 % liegt.

**2.** Farbige kosmetische Zusammensetzung nach Anspruch 1, **dadurch gekennzeichnet, dass** die durchsichtige oder durchscheinende kosmetische Basisformulierung eine in etwa farblose Basisformulierung ist.

**3.** Farbige kosmetische Zusammensetzung nach Anspruch 1 oder 2, **dadurch gekennzeichnet, dass** die durchsichtige oder durchscheinende kosmetische Basisformulierung unter den wässrigen oder öligen Gelen, insbesondere in Stiftform, ausgewählt ist.

**4.** Farbige kosmetische Zusammensetzung nach einem der hervorgehenden Ansprüche, **dadurch gekennzeichnet, dass** die Basisformulierung ein wasserfreies Gel ist, das aus einer bei Umgebungstemperatur flüssigen Fettphase gebildet wird, die polare und/oder apolare Öle enthält und mit einem Gelbildner für die Fettphase strukturiert ist, der unter den hydrophoben pyrogenen Kieselsäuren, gelierenden Polyamiden, hydrophoben Galactomannanen und deren Gemischen ausgewählt ist.

5. Farbige kosmetische Zusammensetzung nach Anspruch 4, **dadurch gekennzeichnet, dass** die gelierenden Polyamide der Formel (I) entsprechen:

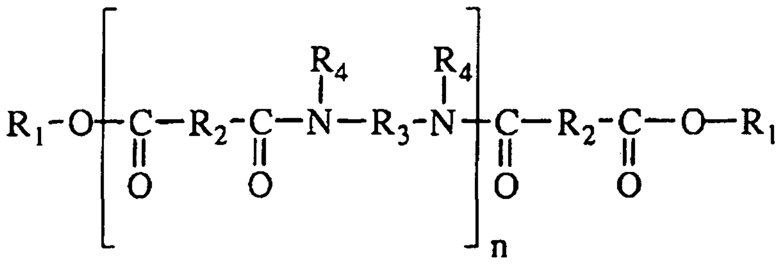 (I),

worin bedeuten: n eine ganze Zahl an Amideinheiten, die so gewählt ist, dass die Anzahl der Estergruppen 10 bis 50 % der Gesamtzahl der Ester- und Amidgruppen ausmacht; die Gruppen $R_1$ jeweils unabhängig voneinander eine Alkyl- oder Alkenylgruppe mit mindestens 4 Kohlenstoffatomen und insbesondere 4 bis 24 Kohlenstoffatomen; die Gruppen $R_2$ jeweils unabhängig voneinander eine Kohlenwasserstoffgruppe mit 4 bis 42 Kohlenstoffatomen, mit der Maßgabe, dass 50 % der Gruppen $R_2$ eine $C_{30-42}$-Kohlenwasserstoffgruppe bedeuten; die Gruppen $R_3$ jeweils unabhängig voneinander eine organische Gruppe, die mindestens zwei Kohlenstoffatome, Wasserstoffatome und gegebenenfalls ein oder mehrere Sauerstoff- oder Stickstoffatome aufweist; und die Gruppen $R_4$ jeweils unabhängig voneinander ein Wasserstoffatom, eine $C_{1-10}$-Alkylgruppe oder eine direkte Bindung zur Gruppe $R_3$ oder zu einer anderen Gruppe $R_4$, sodass das Stickstoffatom, an das die Gruppen $R_3$ und $R_4$ gebunden sind, Teil einer heterocyclischen Struktur ist, die durch $R_4$-N-$R_3$ definiert ist, wobei mindestens 50 % der Gruppen $R_4$ ein Wasserstoffatom bedeuten.

6. Farbige kosmetische Zusammensetzung nach einem der vorhergehenden Ansprüche, **dadurch gekennzeichnet, dass** das oder die Farbmittel unter den wasserlöslichen Farbstoffen, den fettlöslichen Farbstoffen, Pigmenten, Perlglanzpigmenten, Lacken und deren Gemischen ausgewählt ist (sind).

7. Farbige kosmetische Zusammensetzung nach Anspruch 6, **dadurch gekennzeichnet, dass** die wasserlöslichen Farbstoffe unter den Extrakten von Sorghum, *Pterocarpus soyauxii, Monascus, Lawsonia inermis, Mercurialis perenis, Helianthus aanus, Impatiens balsamina, Curcuma longa, Phytolacca decandra, Solidago aureus, Juglans regia, Iris germanica, Alkanna tinctoria, Chrozophora tinctoria, Isatis tinctoria* und deren Gemischen ausgewählt sind.

8. Farbige kosmetische Zusammensetzung nach Anspruch 6, **dadurch gekennzeichnet, dass** die fettlöslichen Farbstoffe unter Sudanrot III, Lutein, Chinizaringrün, Alizurol purple SS, Carotinoiden, wie Lycopin, β-Carotin, Bixin oder Capsantein, Derivaten von Rocou, Derivaten von Fuchsin und deren Gemischen ausgewählt sind.

9. Farbige kosmetische Zusammensetzung nach Anspruch 6, **dadurch gekennzeichnet, dass** die Pigmente weiße oder farbige, anorganische oder organische, umhüllte oder nicht umhüllte Pigmente sind, die unter den Dioxiden von Titan, Zirconium oder Cer, Oxiden von Zink, Eisen oder Chrom, Eisenblau, Chromhydrat, Ruß, Ultramarinen, Manganviolett, Manganpyrophosphat und verschiedenen metallhaltigen Pulvern, wie Pulvern von Silber oder Aluminium, und deren Gemischen ausgewählt sind.

10. Farbige kosmetische Zusammensetzung nach Anspruch 6, **dadurch gekennzeichnet, dass** die Perlglanzpigmente unter den mit Titanoxid oder Bismutoxidchlorid beschichteten Glimmerpigmenten und den mit Eisenoxiden, Eisenblau oder Chromoxid oder mit einem gefällten organischen Pigment beschichteten Glimmerpigmenten ausgewählt sind.

11. Farbige kosmetische Zusammensetzung nach Anspruch 6, **dadurch gekennzeichnet, dass** die Lacke unter den Lacken auf der Basis von Cochenille-Karmin, Salzen von Calcium, Barium, Aluminium, Strontium oder Zirconium, sauren Farbstoffen und deren Gemischen ausgewählt sind.

12. Farbige kosmetische Zusammensetzung nach Anspruch 6, **dadurch gekennzeichnet, dass** sie mindestens einen wasserlöslichen oder fettlöslichen Farbstoff enthält, der in der kosmetischen Basisformulierung löslich ist.

**13.** Farbige kosmetische Zusammensetzung nach Anspruch 12, **dadurch gekennzeichnet, dass** sie als Farbmittel ausschließlich einen oder mehrere in der kosmetischen Basisformulierung lösliche Farbstoffe enthält und dass keine unlöslichen Farbmittel vom Typ der Pigmente, Perlglanzpigmente oder Lacke enthalten sind.

**14.** Farbige kosmetische Zusammensetzung nach Anspruch 12 oder 13, **dadurch gekennzeichnet, dass** die kosmetische Basisformulierung eine lipophile Basisformulierung ist, und **dadurch**, dass sie einen oder mehrere, darin lösliche, lipophile Farbstoffe enthält.

**15.** Farbige kosmetische Zusammensetzung nach einem der vorhergehenden Ansprüche, **dadurch gekennzeichnet, dass** das Farbmittel in einer solchen Menge eingearbeitet wird, dass die Durchlässigkeit einer Schicht der fertigen Zusammensetzung von 10 μm, die bei der Wellenlänge des Maximums eines der Peaks der Absorption oder der Streuung des Farbmittels gemessen wird, im Bereich von 25 bis 80 % liegt.

**16.** Farbige kosmetische Zusammensetzung nach einem der vorhergehenden Ansprüche, **dadurch gekennzeichnet, dass** der Mengenanteil des oder der Farbmittel im Bereich von 0,05 bis 3 Gew.-% und vorzugsweise 0,1 bis 1 Gew.-% Farbmittel, bezogen auf das Gesamtgewicht der farbigen kosmetischen Zusammensetzung, liegt.

**17.** Farbige kosmetische Zusammensetzung nach einem der vorhergehenden Ansprüche, **dadurch gekennzeichnet, dass** die Zusammensetzung in Form eines wasserfreien Lippenstifts oder eines wasserfreien Make-ups vorliegt.

**18.** Verfahren zur Herstellung einer durchsichtigen oder durchscheinenden, farbigen kosmetischen Zusammensetzung nach einem der Ansprüche 1 bis 17, **dadurch gekennzeichnet, dass** es die folgenden Schritte umfasst:

(a) Auswahl einer durchsichtigen oder durchscheinenden kosmetischen Basisformulierung,
(b) Herstellung einer Reihe von Proben dieser durchsichtigen oder durchscheinenden kosmetischen Basisformulierung mit steigenden Mengen eines in der kosmetischen Basisformulierung gelösten oder dispergierten Farbmittels,
(c) Verteilen jeder auf diese Weise hergestellten Probe auf einem durchsichtigen Objektträger, der eine Ausnehmung mit einer Tiefe von 10 μm aufweist,
(d) gegebenenfalls Bearbeiten der überschüssigen Probe auf Bündigkeit, um eine regelmäßige Schicht mit einer Dicke von 10 μm zu erhalten,
(e) Bestimmen der Durchlässigkeit der Schicht für jede Probe bei einer Wellenlänge, die dem Maximum eines der Peaks der Absorption oder der Streuung des Farbmittels entspricht,
(f) Aufnehmen der Eichkurve für die Durchlässigkeit = f (Konzentration des Farbmittels), und
(g) Einbringen mindestens eines Farbmittels in eine durchsichtige oder durchscheinende kosmetische Basisformulierung, die der in Schritt (a) gewählten Basisformulierung entspricht oder davon verschieden ist und die im flüssigen Zustand vorliegt, jeweils in einer Menge, die gemäß der für jedes Farbmittel erstellten Eichkurve eine Durchlässigkeit (bei 10 μm) im Bereich von 20 bis 80 % und vorzugsweise 25 bis 80 % ergibt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 985234 A
- US 3645705 A
- US 3148125 A
- US 5500209 A
- US 5783657 A